# EUROPÄISCHE PATENTSCHRIFT

(11) **EP 1 415 980 B1**
(45) Veröffentlichungstag und Bekanntmachung des Hinweises auf die Patenterteilung: **04.10.2006**
(21) Anmeldenummer: 03023849.7
(22) Anmeldetag: 21.10.2003
(51) Int. Cl.: C07C 251/20, C07C 251/12, C08C 19/28

(54) **Alterungsschutzmittel für Kautschukvulkanisate**
Anti-aging agent for rubber vulcanisates
Agent anti-vieillissement pour vulcanisats de caoutchouc

(30) Priorität: 31.10.2002 DE 10250709
(43) Veröffentlichungstag der Anmeldung: 06.05.2004
(73) Patentinhaber: LANXESS Deutschland GmbH, 51369 Leverkusen (DE)
(72) Erfinder: Oberthür, Markus, Dr., 25541 Brunsbüttel (DE)

(56) Entgegenhaltungen:
- DE-A- 10 022 950
- GB-A- 1 035 262
- DATABASE CA [Online] CHEMICAL ABSTRACTS SERVICE, COLUMBUS, OHIO, US; SUMITOMO CHEMICAL CO., LTD., JAPAN: "N-(1-Methylalkylidene)arylamines" retrieved from STN Database accession no. 99:139477 XP002267366 -& JP 58 099454 A (SUMITOMO CHEMICAL CO., LTD., JAPAN) 13. Juni 1983 (1983-06-13)
- MISRA, VINAY S. ET AL: "Possible antituberculous compds. IX. Prepn. of benzylidene and benzyl derivs. of 4-aminodiphenylamine" J. INDIAN CHEM. SOC. (1960), 37, 481-2, XP008025987
- BARMETTLER, PETER ET AL: "Acid-catalyzed [3,3]-sigmatropic rearrangements of N-propargylanilines" HELVETICA CHIMICA ACTA (1990), 73(6), 1515-73 , XP008026042
- AL-DILAIMI, SOBHI K. ET AL: "Apparent stability constants and thermodynamic functions of cinnamylideneaniline Schiff base complexes with aluminum chloride" THERMOCHIMICA ACTA (1986), 97, 267-70 , XP008026038

## Beschreibung

Die vorliegende Erfindung betrifft Alterungsschutzmittel, basierend auf konjugierte Azadiengruppen enthaltenden organischen Verbindungen, die Kautschukvulkanisate langfristig z.B. gegen thermische Alterung, Ermüdung sowie Alterung durch Sauerstoffeinfluss zu schützen vermögen. Die erfindungsgemäßen Alterungsschutzmittel zeichnen sich weiterhin dadurch aus, dass sie praktisch kaum durch Wasser, Öle und/oder Benzine oder Hydraulikflüssigkeiten aus den Vulkanisaten extrahiert werden.

Es ist bekannt, Kautschukvulkanisate durch Alterungsschutzmittel vor zerstörenden Umwelteinflüssen zu schützen. So werden z.B. zur Verbesserung der Hitze- und Lagerstabilität von Kautschukvulkanisaten phenolische, aminische, schwefelhaltige oder phosphorhaltige Alterungsschutzmittel zugegeben. Einen Überblick über bekannte Alterungsschutzmittel für Kautschuke findet sich beispielsweise in Ullmanns Enzyklopädie der technischen Chemie, Verlag Chemie, Weinheim, 4. Auflage (1974), Band 8, S. 19-45.

GB 1 035 262 beschreibt gewisse N-Alkyl-N'-phenyl-p-phenylendiamine als stabilisatoren von Kautschuk.

Verbesserte, covulkanisierbare Alterungsschutzmittel, die durch Umsetzung von gegebenenfalls substituierten p-Phenylendiaminen und/oder sterisch gehinderten Phenolen mit bifuktionellen Alkyl-, Aryl- und/oder Aralkylverbindungen und anschließender Reaktion des so erhaltenen Produkts mit Schwefel und/oder schwefelliefemden Verbindungen hergestellt werden können, werden in der Deutschen Patentanmeldung DE-A 10 022 950 beschrieben.

In dieser Patentveröffentlichung wird auch eine Zusammenschau über bislang bekannte Alterungsschutzmittel für Kautschukvulkanisate gegeben, wobei insbesondere darauf hingewiesen wird, dass die bekannten Alterungsschutzmittel erhebliche Nachteile besitzen aufgrund der leichten Flüchtigkeit oder Extrahierbarkeit der be-kannten Alterungsschutzmittel. Durch die in DE-A 10 022 950 beschriebenen covulkanisierbaren Alterungsschutzmittel werden diese Nachteile vermieden. Zur Herstellung der dort offenbarten Alterungsschutzmittel ist jedoch eine relativ aufwendige zweistufige Synthese erforderlich, die die Reaktion von beispielsweise Phenylendiaminen mit difunktionellen Alkyl-, Aryl- oder Aralkylverbindungen beinhaltet mit anschließender Umsetzung der bei dieser Reaktion erhaltenen Produkte mit Schwefel und/oder schwefelliefernden Verbindungen.

Es ist daher Aufgabe der vorliegenden Erfindung ein Alterungsschutzmittel zur Verfügung zu stellen, das in eleganter, einstufiger Synthese herstellbar ist und die bereits in DE-A 10 022 950 erwähnten Nachteile, wie Flüchtigkeit und Extrahierbarkeit, der bekannten Alterungsschutzmittel vermeidet, ohne dass die Wirksamkeit des erfindungsgemäßen Alterungsschutzmittels gegenüber den bisher bekannten Alterungsschutzmitteln vermindert ist.

Gegenstand der vorliegenden Erfindung ist die Verwendung von konjugierten Azadiengruppen-enthaltenden organischen Verbindungen der nachstehenden Formeln 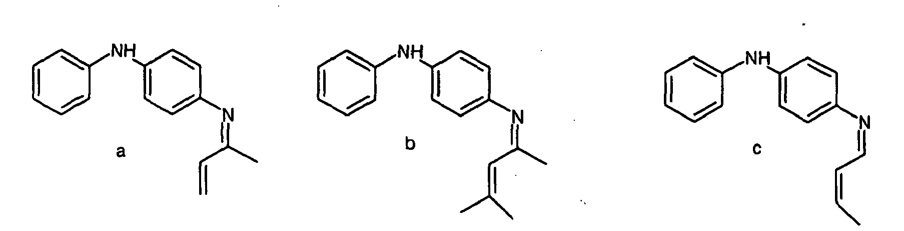 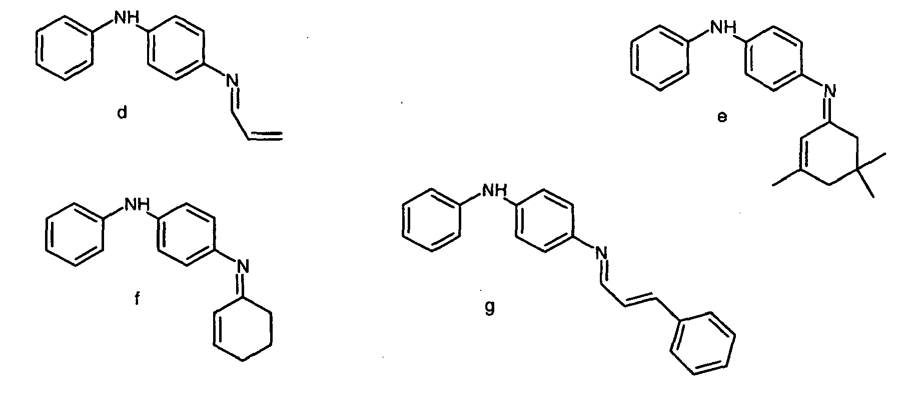 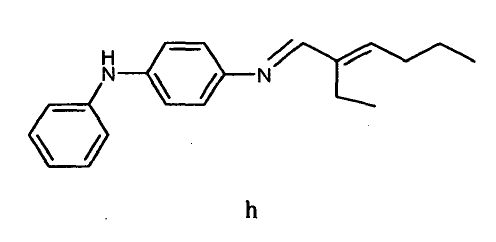 als Alterungsschutzmittel zur Herstellung von Kautschukvulkanisaten aller Art.

Insbesondere sind als konjugierte Azadiengruppen enthaltende organische Verbindungen solche der nachstehenden Formeln zu nennen: 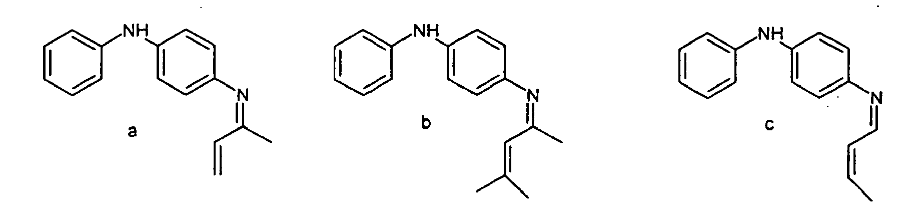 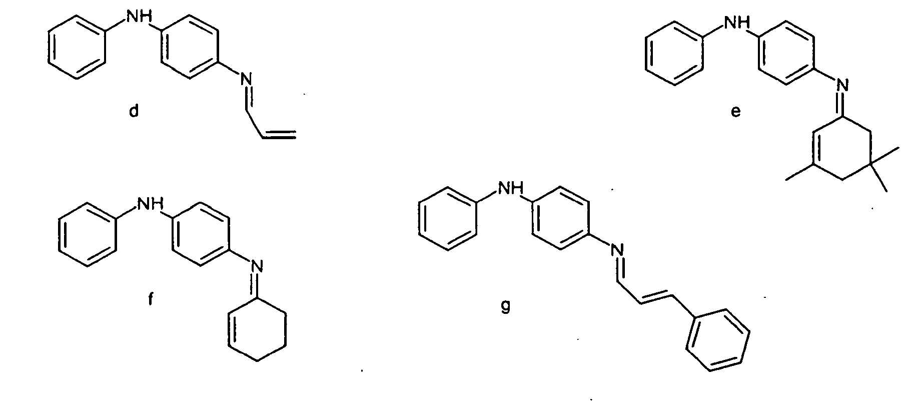
(a) 1-N-(4'-N'-Phenylamino-1'-phenyl)-2-methyl-1-azabuta-1,3-dien,
(b) 1-N-(4'-N'-Phenylamino-1'-phenyl)-2,4-dimethyl-1-azapenta-1,3-dien,
(c) 1-N-(4'-N'-Phenylamino-1'-phenyt)-1-azarenta-1,3-dien,
(d) 1-N-(4'-N'-Phenylamino-1'-phenyl)-1-azabuta-1,3-dien,
(e) 1-N-(4'-N'-Phenylamino-1'-phenyl)-3,5,5-trimethyl-1-azamethylencyclohex-2-en,
(f) 1-N-(4'-N'-Phenylamino-1'-phenyl)-1-azamethylencydohex-2-en,
(g) 1-N-(4'-N'-Phenylamino-1'-phenyl)-4-phenyl-1-azabuta-1,3-dien.

Die erfindungsgemäßen Alterungsschutzmittel können hergestellt werden durch Umsetzung von gegebenenfalls substituierten, primären aromatischen Aminen und gegebenenfalls substituierten konjugierten 1,3-Enonen und/oder 1,3-Enalen und/oder deren synthetischen Äquivalenten, wie deren Halbacetale, Acetale, Aminale, Aza- oder Thiocarbonylderivate.

Namentlich ist folgendes aromatisches Amin genannt: N-Phenyl-4-phenylendiamin.

Beispielsweise werden solche einfach oder mehrfach ungesättigte Carbaldehyde und/oder Ketone der Formel (III) bei der Herstellung der erfindungsgemäßen Alterungsschutzmittel eingesetzt, die nachfolgend genannt werden: Cyclohex-2-en-1-on, 2-Methylpent-2-en-4-on, Prop-1-en-3-al, But-1-en-3-on, But-2-en-1-al, 3,5,5-Trimethylcyclohex-2-en-1-on, 3-Phenylprop-2-enal.

Insbesondere seien z.B. folgende konjugierte Ketone und Aldehyde genannt: 2-Methylpent-2-en-4-on, But-1-en-3-on, 3,5,5-Trimethylcyclohex-2-en-1-on, Prop-2-en-1-al.

Beispielsweise werden solche Derivate einfach oder mehrfach ungesättigter Carbaldehyde und/oder Ketone der Formel (IV) bei der Herstellung der erfindungsgemäßen Alterungsschutzmittel eingesetzt, die nachfolgend genannt werden: 2-Methylpent-2-en-4-thion, But-1-en-3-thion, 3,5,5-Trimethylcyclohex-2-en-1-thion, 2-Methylpent-2-en-4-imin, But-1-en-3-imin, 3,5,5-Trimethylcyclohex-2-en-1-imm, 3,3-Dimethoxybuten, 2,2-Dimethoxy-4-methylpent-3-en, 3,3-Ethylendioxybuten, 2,2-Ethylendioxy-4-methylpent-3-en, 1,1-Dimethoxyprop-2-en, 2,2-Dimethoxybut-3-en, 1,1-Ethylendioxyprop-3-en, 1,1-Dimethoxy-3,5,5-trimethylcyclohex-2-en, 1,1-Diethoxy-3,5,5-trimethylcyclohex-2-en, 1,1-Ethylendioxy-3,5,5-trimethylcyclohex-2-en, 1,1-Dimethoxy-cyclohex-2-en, 1,1-Diethoxy-cyclohex-2-en, 1,1-Ethylendioxy-cyclohex-2-en.

Selbstverständlich können die oben genannten Verbindungen auch im Gemisch miteinander eingesetzt werden.

Das gleiche gilt für die gegebenenfalls einzusetzende Mischung an konjugierten Ketonen und/oder Aldehyden und/oder deren Derivaten wie z.B. Acetalen.

Die Umsetzung des p-Phenylendiamins mit den konjugierten Ketonen und/oder Aldehyden und/oder deren Derivaten wie z.B. Acetalen, wird in Gegenwart von inerten, organischen Lösungsmitteln durchgeführt.

Als inerte, organische Lösungsmittel kommen beispielsweise in Betracht:
Aliphatische oder aromatische Kohlenwasserstoffe, die gegebenenfalls substituiert sein können mit Alkyl-, Alkoxy-, Halogen-, Nitro- Amino-, Sulfogruppen, sowie aliphatische oder aromatische Ether, Amine und Sulfide.

Bevorzugt werden als Lösungsmittel eingesetzt:
Alkylbenzole, Toluol, Xylol, Cymol, Benzine, Chlorbenzol, Dichlorbenzol, Chlortoluol.

Selbstverständlich kann die erwähnte Umsetzung auch ohne Lösungsmittel durchgeführt werden, beispielsweise in einem Überschuss an in flüssiger Form vorliegenden konjugierten Ketonen und/oder Aldehyden und/oder deren Derivaten.

Die günstigste Menge an einzusetzendem Lösungsmittel kann leicht durch entsprechende Vorversuche ermittelt werden.

Üblicherweise wird die Reaktion zur Herstellung des erfindungsgemäßen Alterungsschutzmittels bei Temperaturen von 30°C bis 300°C, bevorzugt bei 120 bis 220°C, besonders bevorzugt bei 150 bis 200°C durchgeführt.

Selbstverständlich kann die Herstellung des erfindungsgemäßen Alterungsschutzmittels durch die zuvor beschriebenen Umsetzungen durch geeignete Katalysatoren beschleunigt werden. Als geeignete Katalysatoren kommen beispielsweise in Betracht:
Lewissäuren, wie z.B. Aluminium- Zink- Zinn- Titan- Eisen oder Borhalogenide, Brönstedtsäuren wie z.B. Schwefel- und Sulfonsäuren, Salzsäure, Phosphorsäure oder auch die in DE 29 01 863 A1 beschriebenen Katalysatoren, wie CaHPO₄ und Hydroxylapatit.

Die Katalysatoren werden in den üblichen Mengen (0,1 bis 10 mol-%, bezogen auf ein Mol aromatisches Amin, eingesetzt.

Die erfindungsgemäßen Alterungsschutzmittel können selbstverständlich auch in Kombination mit bekannten Alterungsschutzmitteln in Kautschukvulkanisaten eingesetzt werden.

Als solche Alterungsschutzmittel sind beispielsweise zu nennen: N-Isopropyl-N'-phenyl-p-phenylendiamin, N-(1,3-Dimethylbutyl)-N'-phenyl-p-phenylendiamin, N-1-Isopropyliden-N'-phenyl-p-phenylendiamin, N-(1,3-Dimethyl-1-butyliden)-N'-phenyl-p-phenylendiamin, N, N'-Di(1,4-dimethylpentyl)-p-phenylendiamin, Phenyl-1-Naphthylamin, octyliertes Diphenylamin, 2,2'-Methylen-bis-(4-methyl-6-tert.-butyl-phenol) sowie 2,2,4-Trimethyl-1,2-dihydrochinolin (monomer und/oder oligomerisiert und/oder polymerisiert), insbesondere N-Isopropyl-N'-phenyl-p-phenylendiamin, N-(1,3-Dimethylbutyl)-N'-phenyl-p-phenylendiamin und N,N'-Di(1,4-dimethylpentyl)-p-phenylendiamin, sowie deren Mischungen untereinander.

Das günstigste Mischungsverhältnis kann dabei leicht durch entsprechende Vorversuche ermittelt werden und richtet sich nach dem jeweiligen Verwendungszweck des zu schützenden Vulkanisats. Üblicherweise beträgt das Mischungsverhältnis vom erfindungsgemäßen Alterungsschutzmittel zu dem bekannten Alterungsschutzmittel 10:1 1 bis 1:10, bevorzugt 5:1 bis 1:2.

Darüber hinaus ist es möglich die erfindungsgemäßen Alterungsschutzmittel mit bekannten Ozonschutzmitteln abzumischen, um einen verbesserten Ozonschutz der Kautschukvulkanisate zu erhalten. Auch hierbei kann der Fachmann das günstigste Mischungsverhältnis je nach dem Verwendungszweck des Kautschukvulkanisats leicht durch Vorversuche ermitteln. Üblicherweise werden den erfindungsgemäßen Alterungsschutzmitteln die Ozonschutzmittel in Mengen von 10:1 1 bis 1:10, bevorzugt 3:1 bis 1:2, bezogen auf das erfindungsgemäße Alterungsschutzmittel, zugegeben. Als zuzusetzende Ozonschutzmittel sind insbesondere zu nennen: N-Isopropyl-N'-phenyl-p-phenylendiamin, N, N'-Di(1,4-dimethylpentyl)-p-phenylendiamin, sowie die in D. Brück, Kautschuk, Gummi, Kunststoffe, 9 (1989) 760-770 detailliert aufgeführten Verbindungen.

Die erfindungsgemäßen Alterungsschutzmittel werden üblicherweise in Mengen von 0,5 Gew.-% bis 10 Gew.-%, bevorzugt 1 Gew.-% bis 5 Gew.-%, bezogen auf 100 Teile des eingesetzten Kautschuks, eingesetzt.

Die Kautschukmischung kann selbstverständlich noch weitere Kautschukhilfsprodukte enthalten, wie Reaktionsbeschleuniger, Wärmestabilisatoren, Lichtschutzmittel, Verarbeitungshilfsmittel, Weichmacher, Tackifier, Treibmittel, Farbstoffe, Pigmente, Wachse, Streckmittel, organische Säuren, Verzögerer, Metalloxide sowie Aktivatoren wie Triethanolamin, Polyethylenglykol, Hexantriol, die in der Gummiindustrie bekannt und üblich sind. Die Kautschukhilfsmittel werden in üblichen Mengen zugemischt und richten sich nach dem jeweils beabsichtigten Verwendungszweck. Übliche Mengen sind beispielsweise Mengen von 0,1 bis 50 Gew.-%, bezogen auf die Gesamtmenge des eingesetzten Kautschuks.

Neben den zuvor erwähnten Hilfsprodukten können den Kautschukmischungen die bekannten Vernetzer zugegeben werden, wie Schwefel oder Schwefelspender, und Vulkanisationsbeschleuniger, wie Mercaptobenzthiazole, Benzthiazolsulfenamide, Guanidine, Thiurame, Dithiocarbamate, Thioharnstoffe und/oder Thiocarbonate. Die Vulkanisationsbeschleuniger und die erwähnten Vernetzer werden üblicherweise in Mengen von 0,1 bis 10 Gew.-%, bevorzugt 0,1 bis 5 Gew.-%, bezogen auf die Gesamtmenge des jeweils eingesetzten Kautschuks, eingesetzt.

Die Vulkanisation der Kautschukmischungen, enthaltend die erfindungsgemäßen Alterungsschutzmittel, kann bei üblichen Temperaturen von 100 bis 200°C, bevorzugt 130 bis 180°C (gegebenenfalls unter Druck 10 bis 300 bar), erfolgen.

Die weitere Abmischung der Kautschuke mit den anderen erwähnten Kautschukhilfsprodukten, Vemetzem und Beschleunigern kann in üblicher Weise mit Hilfe von geeigneten Mischaggregaten, wie Walzen, Innenmischer und Mischextruder, durchgeführt werden.

Die erhaltenen Kautschukmischungen können ggf. auf übliche Weise compoundiert und vulkanisiert werden, wie beispielsweise näher in Encyclopedia of Polymer Science and Engineering, Vol. 4, S. 66 ff (Compoundierung) und Vol. 17, S. 666 ff (Vulkanisation) beschrieben wird.

### Beispiele

Herstellung der erfindungsgemäßen Alterungsschutzmittel

### Verbindung A

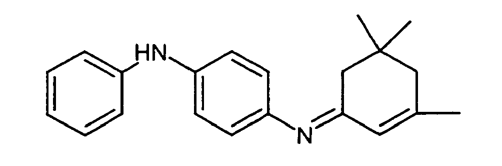

In einem 1 l Vierhalskolben mit Wasserabscheider und Thermometer wurden 184 g (1 mol) 4-ADPA (4-Aminodiphenylamin) in 600 ml Xylol unter Rühren gelöst und mit 2 g p-Toluolsulfonsäure zum Rückfluss erhitzt. 179,7 g (1,3 mol) Isophoron wurden zudosiert. Innerhalb von 8 h spalteten sich 16,5 ml Wasser ab. Anschließend wurden dem Reaktionsgemisch 500 ml Wasser und 50 g NaHCO₃ zugesetzt, gerührt, die Phasen im Scheidetrichter getrennt und die organische Phase über 50 g Na₂SO₄ filtriert. Nach Destillation (0,2 mbar, 100°C) der flüchtigen Anteile wurde das Rohprodukt (schwarz-brauner Rückstand) aus 600 ml Toluol und 300 ml n-Hexan umkristallisiert. Ausbeute: 99 g gelbes, kristallines N-Phenyl-N'-3,3,5-trimethylcyclohex-2-enylen-p-phenylendiamin, Fp.: 126-128°C.

### Verbindung B

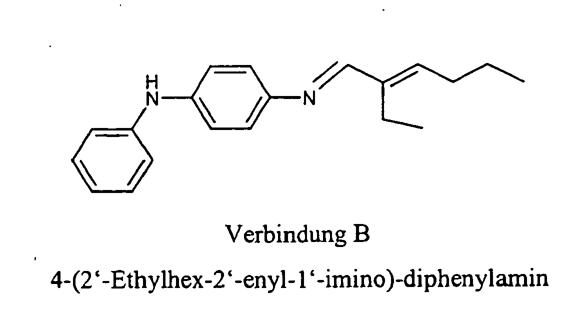

In einem 21 Vierhalskolben mit Wasserabscheider, Thermometer und KPG-Rührer wurden unter Stickstoffatmosphäre 184 g (1 mol) 4-Aminodiphenylamin (4-ADPA), 4 g Eisessig als Katalysator und 600 ml Toluol vorgelegt und zum Rückfluss erhitzt. Bei dieser Temperatur wurden unter Rühren im Laufe von sechs Stunden langsam 163,8 g (1,3 mol) 2-Ethylhex-2-enal zugetropft, wobei sich 17,8 ml Reaktionswasser bildeten und mit dem Wasserabscheider entfernt wurden. Bei gleicher Temperatur ist noch drei Stunden nachgerührt worden, bis eine Dünnschichtkontrolle den vollständigen Verbrauch von 4-ADPA anzeigte. Nach dem Abkühlen auf Raumtemperatur wurde eine Lösung von 3 g NaOH in 100 ml Wasser zugesetzt, um die Säure zu neutralisieren. Im Scheidetrichter erfolgte die Phasentrennung. Das Lösungsmittel ist im Wasserstrahlvakuum entfernt, der Rückstand über eine kurze Vigreux-Kolonne destilliert worden (220°C, 1 mbar). Als Destillat erhält man 283 g eines schwarzbraunen Öls, das mit Hilfe der GC-MS charakterisiert wurde.
GC-MS: Isomerengemisch, 85%ig rein, M/z: 292 (M+), 277, 263, 249, 234, 184, 183 (100%), 167.

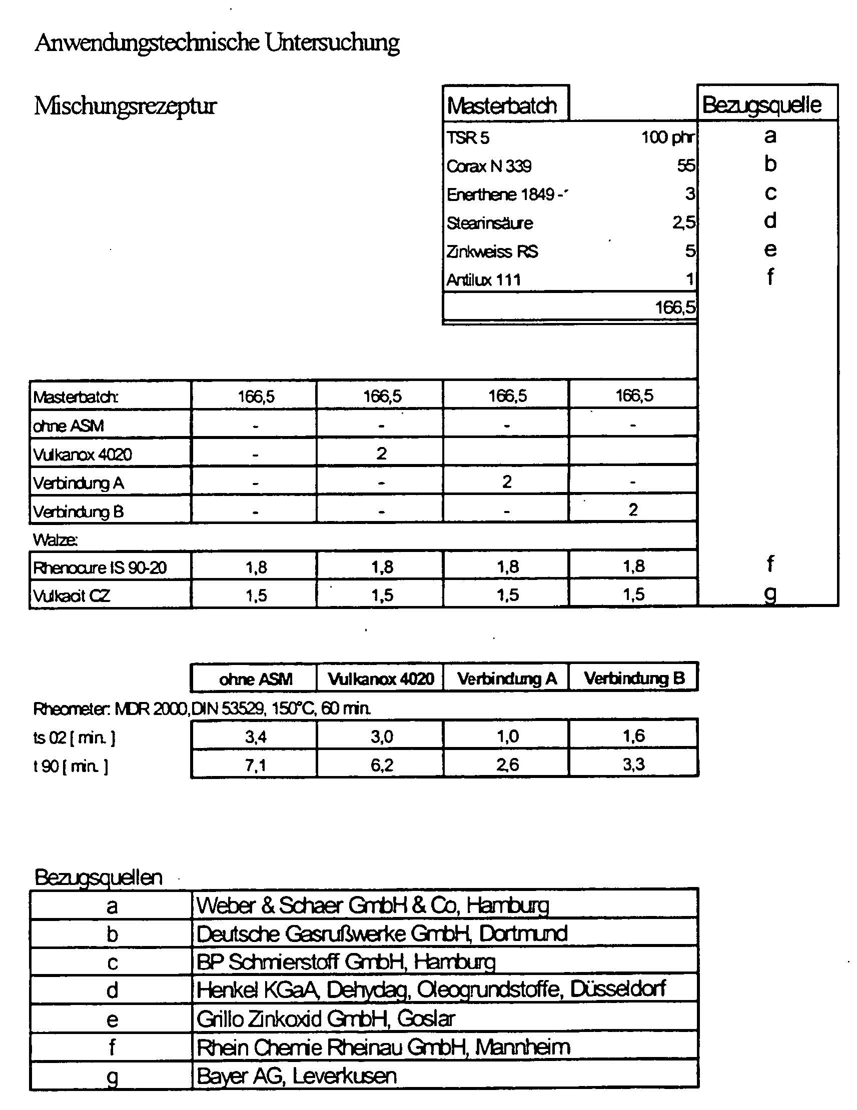

### Die Kautschukmischungen wurden wie folgt hergestellt:

In einem TPE-Kneter GK 1,5 E (Volumen ca.1500 ml) Temperatur = 40°C wurden alle angeführten Substanzen, außer Schwefel und Vulkacit ® CZ in die Kautschukmatrix eingemischt. Anschließend wurden Schwefel (Rhenocure 95-2) und Vulkacit ® CZ auf einer Walze (150 mm Ø) bei 40°C in die Mischung eingebracht. Die Drehzahl der Walzen betrug 12 U/min bei einer Friktion von 1,22.

Die Vulkanisation der Mischungen zu Gummiplatten (100 x 100 x 2 mm) erfolgte danach in elektrischen Heizpressen (300 bar) bei 150°C bis t₉₀+ 5 min. der Rheometerkurven.

Die Vulkanisate wurden unter verschiedenen Bedingungen auf ihre mechanischdynamischen Eigenschaften geprüft:
1. im frischen Zustand
2. nach sieben Tagen Heißluftalterung bei 70°C
3. nach 14 Tagen Heißluftalterung
4. nach Extraktion mit saurem Wasser bei pH 4 und 80°C

Die Heißluftalterung der Probekörper soll den normalen Alterungsprozess von Gummiartikeln in verkürzter Zeit simulieren und ist dem Fachmann zu diesem Zweck wohlbekannt.

Die Extraktion im sauren Wasser bei pH 4 soll bei diesen Tests beispielsweise die Einwirkung von saurem Regenwasser simulieren und die aminischen Alterungsschutzmittel unter verschärften Bedingungen aus dem Probekörper herauslösen.

Die Extraktion wurde durch Immersion der Probekörper in temperierte (80°C) wässrige Pufferlösung bewerkstelligt. Das Extraktionsmedium wurde täglich erneuert.

Als Referenz wurden Probekörper mitgetestet, die kein Alterungsschutzmittel enthielten (ohne ASM) und solche, die mit Vulkanox® 4020 (6-PPD), dem Stand der Technik, gegen Alterungsprozesse geschützt waren.

Die mechanisch-dyriamischen Eigenschaften, z. B. Modul [M], Zugfestigkeit [ZF] und Bruchdehnung [BD] der Vulkanisate sind in den nachfolgenden Tabellen aufgeführt.

**Zugversuche nach DIN 53504, Normstab S 2 bei 20 °C**

| Probekörper frisch vulkanisiert | | o**hne ASM** | **Vulkanox 4020** | **Verbindung A** | **Verbindung B** |
|---|---|---|---|---|---|
| M 100 [%] | MPa | 3,1 | 3,0 | 2,3 | 2,7 |
| M 300 [%] | MPa | 16,4 | 16,1 | 12,5 | 13,6 |
| ZF | MPa | 28,7 | 29,6 | 29,5 | 28,7 |
| BD | % | 492 | 507 | 600 | 553 |

**Alterung ohne Extraktion**

| 7 Tage Heißluftalterung, 70°C | | **ohne ASM** | **4020** | **Verbindung A** | **Verbindung B** |
|---|---|---|---|---|---|
| M 100 [%] | MPa | 3,7 | 3,8 | 3,2 | 3,9 |
| M 300 [%] | MPa | 17,6 | 18,5 | 16,0 | 18,1 |
| ZF | MPa | 22,7 | 26,5 | 27,8 | 28,0 |
| BD | % | 383 | 437 | 504 | 482 |
| | | | | | |

| 14 Tage Heißluftalterung, 70°C | | **ohne ASM** | **4020** | **Verbindung A** | **Verbindung B** |
|---|---|---|---|---|---|
| M 100 [%] | MPa | 4,3 | 4,4 | 3,4 | 4,0 |
| M 300 [%] | MPa | 19,2 | 20,3 | 15,8 | 17,1 |
| ZF | MPa | 23,7 | 29,1 | 26,5 | 26,7 |
| BD | % | 371 | 424 | 488 | 442 |

**Nach Extraktion in Wasser, 80°C, pH 4**

| 7 Tage Extraktion | | **ohne ASM** | **4020** | **Verbindung A** | **Verbindung B** |
|---|---|---|---|---|---|
| M 100 [%] | MPa | 3.8 | 3,9 | 3,2 | 3.6 |
| M 300 [%] | MPa | 20,0 | 21,3 | 15,8 | 17.1 |
| ZF | MPa | 23,9 | 28,1 | 27,8 | 25,9 |
| BD | % | 347 | 378 | 504 | 482 |

### Ergebnisse der Testreihen:

Es zeigte sich bei diesen Tests, dass die erfindungsgemäßen Verbindungen A und B in den mechanischen Eigenschaften bereits im frischen, ungealterten und nichtextrahierten Probekörper bessere Bruchdehnungswerte als der Standard aufwiesen (600 % bei **A** bzw 553 % bei **B** gegenüber 507 % bei Vulkanox® 4020) und insofern den Gummiartikel besser gegen Alterungseinflüsse zu schützen vermögen.

Dieses Ergebnis wiederholte sich auch nach sieben und nach vierzehn Tagen Heißluftalterung bei 70°C.

Noch stärker ausgeprägt ist der Unterschied zum Standard nach Lagerung der Probekörper unter extraktiven Bedingungen in saurem Wasser. Der mit Vulkanox® 4020 geschützte Gummikörper erreicht nur noch eine Bruchdehnung von 378 %, während der Schutz durch die erfindungsgemäßen Verbindungen Bruchdehnungswerte von 504 % (**A**) und 482 % (**B**) zulässt.

## Patentansprüche

1. Verwendung von konjugierten Azadiengruppen-enthaltenden organischen Verbindungen der nachstehenden Formeln 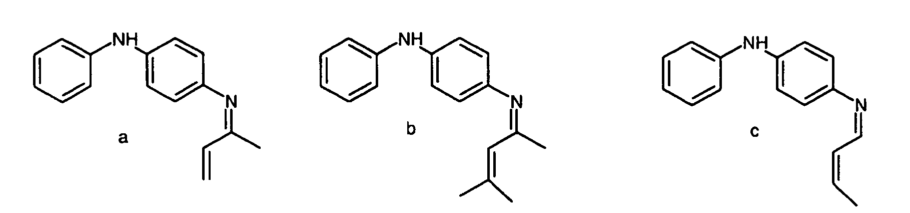 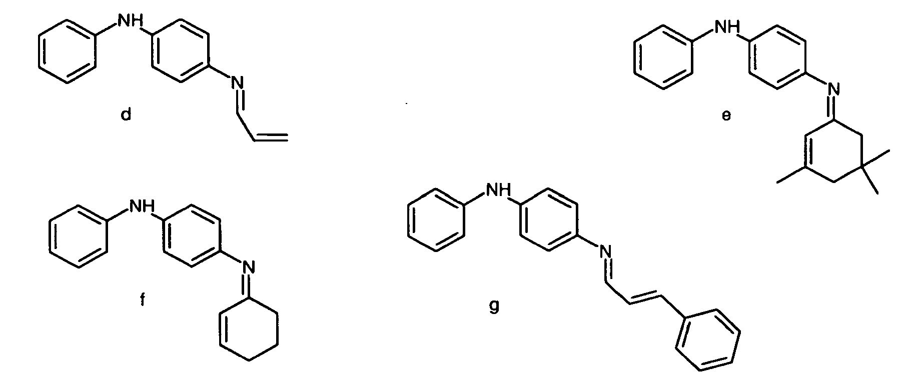 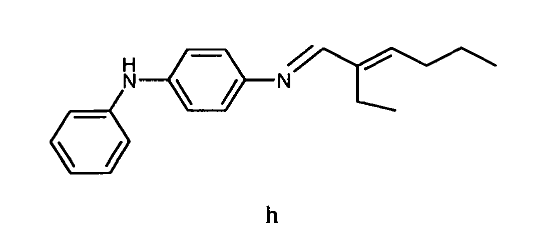 als Alterungsschutzmittel zur Herstellung von Kautschukvulkanisaten aller Art.

## Claims

1. Use of organic compounds comprising conjugated azadiene groups and having the following formulae: 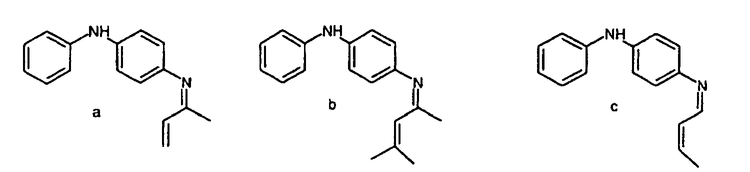 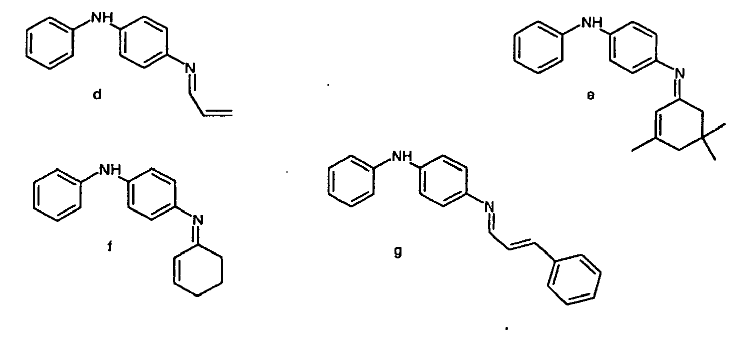 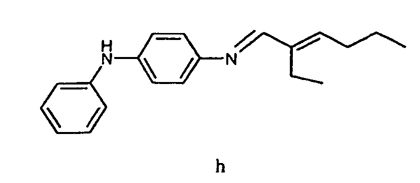 as anti-ageing agents for producing rubber vulcanizates of any kind.

## Revendications

1. Utilisation de composés organiques renfermant des groupes azadiène conjugués de formules suivantes: 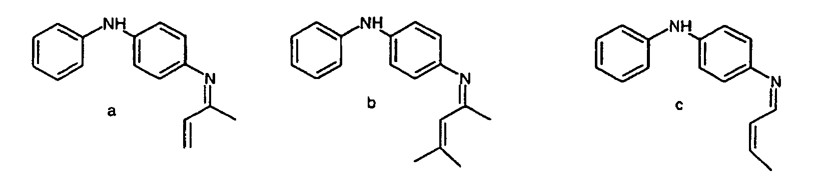 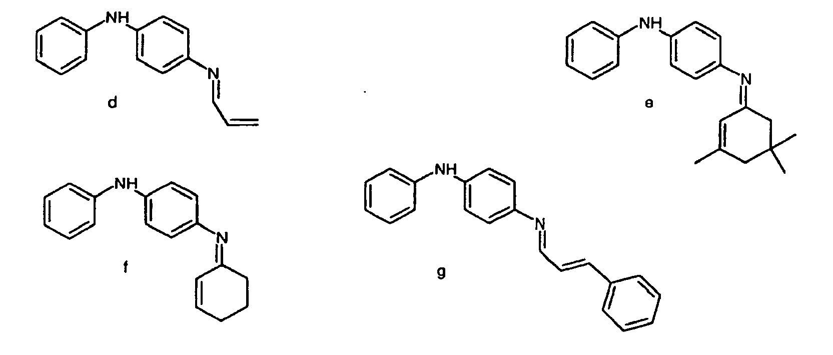 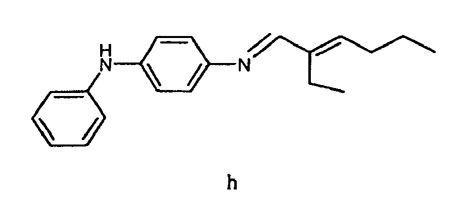 en tant qu'agents anti-vieillissement pour la production de vulcanisats de caoutchouc de tous types.
